# FASCICULE DE BREVET EUROPEEN

(11) **EP 2 416 672 B1**
(45) Date de publication et mention de la délivrance du brevet: **23.01.2013**
(21) Numéro de dépôt: 10713328.2
(22) Date de dépôt: 06.04.2010
(51) Int. Cl.: A23L 2/48, A23L 2/50, A23L 3/32, C02F 1/48, C12N 13/00

(54) **PROCEDE DE PERMEABILISATION MEMBRANAIRE DE CELLULES BIOLOGIQUES PAR L'UTILISATION D'UN CHAMP ELECTRIQUE PULSE**
VERFAHREN ZUM DURCHLÄSSIGMACHEN DER MEMBRANEN VON BIOLOGISCHEN ZELLEN DURCH VERWENDUNG EINES GEPULSTEN ELEKTRISCHEN FELDS
METHOD FOR RENDERING THE MEMBRANES OF BIOLOGICAL CELLS PERVIOUS BY USING A PULSED ELECTRIC FIELD

(30) Priorité: 07.04.2009 FR 0952262
(43) Date de publication de la demande: 15.02.2012
(73) Titulaire: Commissariat à l'Énergie Atomique et aux Énergies Alternatives, 75015 Paris (FR)
(72) Inventeur: SCHRIVE, Luc, F-30130 Pont Saint Esprit (FR); LUMIA, Guy, F-30130 Pont Saint Esprit (FR); PUJOL, Florian, F-84500 Bollene (FR)
(74) Mandataire: Ilgart, Jean-Christophe
(86) Numéro de dépôt international: PCT/EP2010/054511
(87) Numéro de publication internationale: WO 2010/115875

(56) Documents cités:
- EP-A2- 0 283 700
- FR-A1- 2 792 207
- US-A- 5 048 404
- US-A- 5 514 391
- US-A- 6 093 432
- US-A1- 2004 084 381
- US-A1- 2006 013 927
- MINGYU JIA, Q. HOWARD ZHANG, DAVID B. MIN: "Pulsed electric field processing effects on flavor compounds and microorganisms of orange juice" FOOD CHEMISTRY, [Online] vol. 65, 1999, pages 445-451, XP002560724 DOI: 10.1016/S0308-8146(98)00186-1 Extrait de l'Internet: URL:http://www.sciencedirect.com/science?_ ob=ArticleURL&_udi=B6T6R-3W4B1M2-5&_user=9 87766&_rdoc=1&_fmt=&_orig=search&_sort=d&_ docanchor=&view=c&_searchStrId=1136858377& _rerunOrigin=google&_acct=C000049880&_vers ion=1&_urlVersion=0&_userid=987766&md5=cc0 c88eee3c8509eb30df134ca4f6972> [extrait le 2009-12-16]
- S.F. AGUILAR-ROSAS, M.L. BALLINAS-CASARRUBIAS, G.V. NEVAREZ-MOORILLON, O. MARTIN-BELLOSO, E. ORTEGA-RIVAS: "Thermal and pulsed electric fields pasteurization of apple juice: Effects on physicochemical properties and flavour compounds" JOURNAL OF FOOD ENGINEERING, [Online] vol. 83, 2007, pages 41-46, XP002560725 DOI: 10.1016/j.jfoodeng.2006.12.011 Extrait de l'Internet: URL:http://www.sciencedirect.com/science> [extrait le 2009-12-16]
- CHARLES-RODRIGUEZ, NEVAREZ-MOORILLON, ZHANG, ORTEGA-RIVAS: "COMPARISON OF THERMAL PROCESSINGAND PULSED ELECTRIC FIELDS TREATMENTIN PASTEURIZATION OF APPLE JUICE" FOOD AND BIOPRODUCTS PROCESSING, vol. 85, no. 2, juin 2007 (2007-06), pages 93-97, XP002560726 DOI: 10.1205/fbp06045 Extrait de l'Internet: URL:http://www.sciencedirect.com/science?_ ob=ArticleURL&_udi=B8JGD-4S1T0R1-3&_user=9 87766&_rdoc=1&_fmt=&_orig=search&_sort=d&_ docanchor=&view=c&_searchStrId=1138075031& _rerunOrigin=google&_acct=C000049880&_vers ion=1&_urlVersion=0&_userid=987766&md5=6e8 b40f67282284a956f67ce93e18214> [extrait le 2009-12-16]
- Zsuzsanna Cserhalmi: "Non-Thermal Pasteurization of Fruit Juice Using High Voltage Pulsed Electric Fields"; "Part I: Processing technology - Chapter 6" In: Y. H. Hui: "Handbook of Fruits and Fruit Processing" 10 décembre 2007 (2007-12-10), Blackwell Publishing , XP002560727 ISBN: 9780813819815 , pages 95-114 figure 6.3 et 6.4; tableau 6.2 et 6.3
- C.J. MCDONALD, S.W. LLOYD, M.A. VITALE, K. PETERSSON, F. INNINGS: "Effects of Pulsed Electric Fields on Microorganisms in Orange Juice Using Electric field Strengths of 30 and 50 kV/cm" JOURNAL OF FOOD SCIENCE, vol. 65, no. 6, 2000, pages 984-989, XP002560728 DOI: 10.1111/j.1365-2621.2000.tb09404.x Extrait de l'Internet: URL:http://www3.interscience.wiley.com/jou rnal/119036064/abstract?CRETRY=1&SRETRY=0> [extrait le 2009-12-16]
- Q. ZHANG, G. V. BARBOSA-CANOVAS & B. G. SWANSON: "Engineering Aspects of Pulsed Electric Field Pasteurization" JOURNAL OF FOOD ENGINEERING, vol. 25, 1995, pages 261-281, XP002560729 DOI: 10.1016/0260-8774(94)00030-D Extrait de l'Internet: URL:http://www.sciencedirect.com/science?_ ob=ArticleURL&_udi=B6T8J-3YF4BDT-X&_user=9 87766&_rdoc=1&_fmt=&_orig=search&_sort=d&_ docanchor=&view=c&_searchStrId=1138184232& _rerunOrigin=google&_acct=C000049880&_vers ion=1&_urlVersion=0&_userid=987766&md5=3d7 f6a2b059b06bc4e9267aa3255301d> [extrait le 2009-12-16]

## Description

### DOMAINE TECHNIQUE

La présente invention a trait à un procédé de perméabilisation membranaire de cellules biologiques par l'utilisation d'un champ électrique pulsé.

Ce procédé trouve tout particulièrement son application dans le domaine de la pasteurisation, c'est-à-dire du domaine du traitement des produits alimentaires (tels que le lait, les crèmes, la bière, les jus de fruits) consistant à détruire les microorganismes, notamment pathogènes présents initialement dans ces produits.

Le domaine de l'invention est donc celui de la perméabilisation membranaire de cellules biologiques et, en particulier, de la destruction de cellules biologiques par pasteurisation.

### ÉTAT DE LA TECHNIQUE ANTÉRIEURE

Les techniques de pasteurisation ont fait l'objet de nombreuses études dans l'art antérieur, eu égard notamment à l'essor des produits longue conservation, qui nécessitent une présence très faible de micro-organismes pour pouvoir être consommables à longue durée.

Classiquement, la pasteurisation consiste à chauffer les aliments à une température définie pendant une durée définie de sorte à dépasser le seuil de thermorésistance des bactéries pathogènes étant à l'origine de la détérioration des aliments puis à refroidir rapidement lesdits aliments chauffés (à des températures de 3 à 4°C), de sorte à prévenir la multiplication des bactéries qui n'ont pas été détruites.

Ce principe classique a fait l'objet de nombreuses variantes appartenant à la catégorie des traitements dits « traitements thermiques ».

Les traitements thermiques pour la pasteurisation peuvent consister en l'utilisation comme vecteur de chaleur des moyens suivants :
- les radiations électromagnétiques, telles que les radiations infrarouges, les radiations micro-ondes ;
- la chaleur découlant du phénomène d'effet Joule créée dans un tube à l'intérieur duquel circule le produit à pasteuriser ;
- la chaleur ohmique résultant du passage d'un courant électrique à travers le produit à pasteuriser.

Les températures de pasteurisation atteintes par voie thermique s'échelonnent classiquement de 70°C à 85°C. Toutefois, il peut subsister, après traitement dans ces gammes de températures, certaines formes pathogènes telles que des spores incompatibles pour des produits destinés à l'alimentation.

Pour détruire ces formes pathogènes, une des solutions peut consister à chauffer les aliments à des températures plus élevées que la gamme susmentionnée (par exemple à des températures supérieures à 90°C). Toutefois, l'utilisation de températures plus élevées s'accompagne inéluctablement d'une dénaturation du produit traité, telle qu'une dénaturation des protéines présentes dans le produit, ce qui ne va pas souvent sans une perte des qualités gustatives du produit.

Pour remédier à ces inconvénients, il a été proposé de recourir à des procédés dits « à faible température », de sorte à conserver le goût original du produit. Ces procédés consistent à recourir à des moyens d'élimination des bactéries pathogènes autres que l'utilisation d'un chauffage, permettant de traiter les aliments à des températures ne dépassant pas 60°C. Ces moyens peuvent consister en des rayonnements ionisants, l'utilisation de hautes pressions, en de la lumière pulsée, en l'utilisation d'un gaz tel que le gaz carbonique.

Eu égard de ce qui a déjà été proposé en matière de procédé de perméabilisation membranaire, et en particulier de procédé de pasteurisation, les auteurs se sont proposé de développer un nouveau procédé de perméabilisation membranaire qui permet, notamment, lorsqu'il est mis en oeuvre en vue de pasteuriser un produit alimentaire, d'améliorer le taux de mortalité des cellules indésirables.

La publication dans Food Chemistry, vol. 65, 1999, p.445-451 a trait à une étude sur l'influence de l'utilisation d'un champ électrique pulsé sur les arômes et les microorganismes contenus dans un jus d'orange.

La publication dans le Journal of Food engineering, vol. 83, 2007, p.41-46 a trait à une étude comparée de la pasteurisation d'un jus de pomme par une technique impliquant un champ électrique pulsé et une technique dite thermique.

La publication dans Food and Bioproducts Processing, vol. 85, n°2, p.93-97 a trait à l'étude de l'inactivation d'E. Coli dans un jus de pomme par utilisation d'un champ électrique pulsé.

La publication dans le Handbook of Fruits and Fruit Processing, p.95-114 fait état du traitement d'un jus d'orange mettant en oeuvre un champ électrique pulsé.

### EXPOSÉ DE L'INVENTION

Ainsi, l'invention a trait, selon un premier objet, à un procédé de perméabilisation membranaire de cellules biologiques contenues dans un produit, ledit procédé étant mis en oeuvre dans un dispositif de traitement comprenant au moins une chambre de traitement émettant un champ électrique pulsé, ledit procédé comprenant les étapes suivantes:
- une étape d'alimentation du dispositif de traitement en produit comprenant lesdites cellules biologiques à un débit d'alimentation prédéterminé à partir d'une unité d'alimentation comprenant ledit produit ;
- une étape d'introduction du produit comprenant lesdites cellules biologiques dans ladite chambre de traitement à un débit d'introduction correspondant au débit d'alimentation susmentionné auquel s'ajoute le débit de soutirage mentionné à l'étape de soutirage ci-dessous ;
- une étape de traitement dudit produit introduit dans ladite chambre par un champ électrique pulsé ;
- une étape de soutirage, à un débit de soutirage prédéterminé, du produit en sortie de ladite chambre de sorte à le réacheminer en amont de ladite chambre et en aval de ladite unité d'alimentation.

Il s'entend que le procédé de l'invention est un procédé *in vitro* de perméabilisation membranaire de cellules biologiques, c'est-à-dire qui est mis en oeuvre avec un produit se situant à l'extérieur d'un organisme animal.

Les inventeurs ont constaté, de façon surprenante, que le procédé de l'invention permet d'obtenir une augmentation significative de la perméabilisation membranaire de cellules et ce avec au moins une chambre de traitement (l'augmentation étant quantifiée en terme de taux de mortalité des cellules) par rapport à un procédé comprenant un ou plusieurs passages du produit dans la chambre susmentionnée sans utiliser d'étape de soutirage telle que mentionnée ci-dessus et ce pour une même énergie spécifique utilisée.

Selon l'invention, les cellules traitées dans le cadre du procédé peuvent être des cellules procaryotes, des cellules eucaryotes, ces cellules pouvant être vivantes ou mortes, entières ou partielles et d'origine animale ou végétale.

En particulier, ces cellules peuvent être issues d'organismes unicellulaires ou pluricellulaires, tels que les bactéries (en particulier, les eubactéries), les champignons (en particulier, les moisissures, les levures (comme *Saccharomyces cerevisiae),* les moisissures, les algues (en particulier, les microalgues), les virus et les prions.

Il peut s'agir également d'organites intracellulaires ou de composés internes aux cellules, tels que des mitochondries, des virus, des protéines, des prions.

Les cellules peuvent être en phase végétative ou en phase de dormance (tel que cela peut être le cas des spores, telles que les spores bactériennes).

Les cellules à traiter sont comprises dans un produit, qui peut être un liquide alimentaire, tel qu'un jus de fruits, un jus de légumes, du lait, de l'eau.

En outre, le produit comprenant lesdites cellules peut contenir des tissus, des macromolécules, telles que des biopolymères, des molécules organiques ou inorganiques.

Le champ électrique pulsé auquel est soumis le produit comprenant les cellules a pour effet de perméabiliser lesdites cellules.

Sans vouloir être lié par la théorie, l'exposition d'une cellule à un champ électrique extérieur induit une différence de potentiel électrique de part et d'autre de la membrane constitutive de la cellule. Lorsque ce champ est très intense (notamment lorsqu'il est supérieur à 10 000 V/cm), il peut induire un potentiel transmembranaire de valeur plus élevée que le potentiel naturel de la cellule. Lorsque le potentiel transmembranaire atteint une valeur critique, les phénomènes électrostatiques entre les molécules chargées de part et d'autre de la membrane entraînent la formation de pores dans la membrane cellulaire augmentant ainsi sa perméabilité. Lorsque la formation de pores dans la membrane cellulaire est irréversible, ceci peut entraîner la migration vers l'extérieur du contenu cellulaire et, ainsi, la mort de la cellule.

Selon l'invention, le champ électrique pulsé, en fonction du taux de perméabilisation désirée présentera des caractéristiques nécessaires à l'obtention de cette perméabilisation en termes de valeur de tension, de nombre d'impulsions, de forme du signal, d'énergie spécifique délivrée par ledit champ.

Le champ électrique pulsé se matérialise classiquement sous forme d'impulsions électriques résultant, classiquement, de décharges électriques de durée pouvant s'échelonner de 50 nanosecondes à 1 milliseconde, par exemple 1 µs et délivrant une tension produisant une valeur crête du champ électrique pulsé allant de 5 kV/cm à 200 kV/cm. La tension peut aller de 100 V à 100 000 V.

L'utilisation d'impulsions électriques permet de minimiser le phénomène d'échauffement du produit par effet Joule et ainsi la température de celui-ci (classiquement inférieure à 50°C). Cela permet d'éviter la dénaturation de composants présents dans le produit, laquelle dénaturation est classiquement rencontrée lors de la mise en oeuvre de procédé engageant de hautes températures.

Les impulsions électriques sont délivrées au sein d'une chambre de traitement pouvant se présenter sous forme d'une enceinte comprenant des électrodes qui permettent le passage du courant sous forme d'impulsions électriques. Ces électrodes peuvent être planes, circulaires, co-axiales, co-linéaires, tournantes ou présentant toute autre géométrie appropriée.

Comme annoncé précédemment, le procédé comprend une étape d'alimentation du dispositif de traitement en produit à traiter comprenant lesdites cellules à un débit d'alimentation prédéterminé à partir d'une unité d'alimentation comprenant ledit produit.

Cette étape consiste classiquement à faire transiter le produit à traiter d'une unité d'alimentation, telle qu'un réservoir, où il est contenu vers une conduite d'alimentation laquelle est reliée à la chambre de traitement.

D'un point de vue concret, le produit à traiter alimente le dispositif de traitement à partir d'une unité d'alimentation via une conduite d'alimentation munie éventuellement d'une pompe d'alimentation à un débit d'alimentation prédéterminé, tel qu'un débit s'échelonnant de 0,01 L/h à 10 000 L/h, par exemple un débit de 20 L/h.

Ensuite, le procédé de l'invention comprend une étape d'introduction du produit comprenant lesdites cellules biologiques dans ladite chambre de traitement à un débit d'introduction correspondant au débit d'alimentation susmentionné auquel s'ajoute le débit de soutirage mentionné à l'étape de soutirage ci-dessous.

Une fois le produit introduit dans la chambre de traitement, il est soumis à un champ électrique pulsé se matérialisant par des impulsions électriques, qui vont occasionner une perméabilisation des cellules contenues dans le liquide.

Les impulsions électriques sont avantageusement des impulsions, pouvant s'échelonner de 100 à 100 000 V, par exemple de 10 000 à 50 000 V et délivrant une énergie suffisante pour obtenir la perméabilisation souhaitée selon l'effet biologique recherché. L'énergie délivrée par chaque impulsion est classiquement comprise entre 0,005 J et 500 J.

Ainsi, lorsque l'on souhaite obtenir une perméabilisation des cellules engendrant la mort de ces dernières (notamment en vue d'une pasteurisation ou d'une stérilisation du produit), les paramètres de traitement et, notamment le nombre total d'impulsions délivré à chaque élément de volume (correspondant au volume de la chambre de traitement) seront choisies de sorte à délivrer au produit une énergie spécifique supérieure à 30 MJ/m³, de préférence supérieure à 100 MJ/m³.

Pour obtenir la mort de la cellule, selon l'énergie délivrée à chaque impulsion et selon le volume de la chambre de traitement, le nombre total d'impulsions délivré à chaque élément de volume (correspondant au volume de la chambre de traitement) sera ajusté pour atteindre les valeurs d'énergie spécifique voulues. Ce nombre total d'impulsions peut être compris entre 10 et 1000.

Lorsque l'on souhaite obtenir une perméabilisation des cellules de sorte à accélérer les échanges entre la cellule et le milieu environnant, les impulsions électriques émises par la chambre de traitement peuvent être choisies de sorte à délivrer au produit sur la durée totale du procédé une énergie spécifique inférieure à 20 MJ/m³, par exemple allant de 1 à 20 MJ/m³. Le nombre total d'impulsions peut être compris entre 2 et 100.

Il est entendu que l'homme du métier en fonction du taux de perméabilisation souhaité choisira les caractéristiques des impulsions électriques appropriées en termes de nombre d'impulsions, de tension électrique, d'énergie spécifique délivrée.

Le nombre d'impulsions à appliquer à chaque élément de volume peut être réparti en plusieurs séries.

A titre d'exemple, lorsque l'on considère que 120 impulsions sont nécessaires à chaque élément de volume pour traiter le produit, le nombre total d'impulsions à appliquer pourra être répartie, par exemple, en 3 séries, 6 séries ou 10 séries, chaque série permettant d'appliquer, respectivement, 40, 20 ou 12 impulsions.

Au sein de la chambre de traitement, le produit, notamment lorsqu'il se présente sous forme d'un liquide, peut être soumis à un régime hydraulique turbulent. Ce mode hydraulique est particulièrement avantageux. En effet, la perméabilisation des cellules lors d'une décharge électrique s'effectue préférentiellement sur les faces de la membrane cellulaire situées en vis-à-vis des électrodes. Lorsqu'une cellule est en mouvement (ce qui est le cas lorsque le produit est soumis à un régime turbulent) désordonné par rapport aux électrodes et qu'il est soumis à une série d'impulsions, elle subira des impacts disséminés, ce qui contribuera à augmenter l'efficacité du traitement en terme de perméabilisation. A l'inverse, une cellule immobile par rapport aux électrodes sera soumise à une concentration des impacts, ce qui diminuera l'efficacité du traitement.

Une fois l'étape de traitement réalisée, il est prévu, selon le procédé de l'invention, une étape de soutirage, en aval de la chambre de traitement, du produit traité dans la chambre de traitement à un débit de soutirage prédéterminé, de sorte à réacheminer ce produit soutiré en amont de la chambre de traitement et en aval de ladite unité d'alimentation, moyennant quoi le produit soutiré est de nouveau introduit dans la chambre de traitement et soumis de nouveau au champ électrique pulsé.

Selon un mode particulier de l'invention, l'étape de soutirage est réalisée par le biais d'au moins une boucle de circulation reliant la partie aval de la chambre de traitement à la partie amont de celle-ci. A la sortie de la chambre de traitement, le produit traité est soutiré via l'entrée de boucle de circulation située en aval de la chambre et injecté en amont de la chambre via la sortie de la boucle de circulation, cette boucle de circulation se présentant sous forme d'une conduite de circulation.

Le débit de soutirage peut être supérieur ou égal au débit d'alimentation susmentionné.

Avantageusement, le débit de soutirage est supérieur au débit d'alimentation, par exemple de 2 à 100 fois plus élevé, de préférence de 2 à 20 fois. Cela permet que chaque fraction de produit passe au moins deux fois par la chambre de traitement.

La mise en oeuvre d'une étape de soutirage conforme à l'invention présente les avantages suivants.

Cela permet d'amoindrir le phénomène de court-circuit hydraulique, c'est-à-dire la probabilité qu'une cellule prise sur l'ensemble de la population cellulaire traitée passe plus rapidement dans la chambre de traitement que le reste de cette population.

Pour une énergie spécifique donnée, on obtient, grâce à la mise en oeuvre de l'étape de soutirage conforme à l'invention, de meilleurs résultats en terme de perméabilisation de cellule que pour un procédé présentant un premier traitement du liquide par un passage dans une chambre de traitement suivi d'un second traitement dans la même chambre de traitement après avoir été stocké dans une cuve entre le premier traitement et le second traitement.

Le procédé ne comprend, avantageusement, pas d'étape de stockage stagnant du produit traité avant l'étape de soutirage, c'est-à-dire avant réintroduction de celui-ci dans la chambre de traitement via l'étape de soutirage.

Un autre avantage provient d'un effet inattendu lié au fait que le nombre d'impulsions délivré à chaque élément de volume est délivré en plusieurs fois du fait de l'existence de la boucle de recyclage. Il a été constaté, de façon surprenante, qu'il était plus avantageux, pour un régime hydraulique donné dans la chambre de traitement et un nombre total d'impulsions donné, de répartir ce nombre en plusieurs fois plutôt que de soumettre le produit à traiter au nombre total d'impulsions en une seule fois, sans utiliser de boucle de recyclage.

Selon un autre mode de réalisation de l'invention, l'étape de soutirage peut être réalisée via plusieurs boucles de circulation dont les entrées sont situées en aval de la chambre de traitement et dont les sorties sont situées en amont de la chambre de traitement.

Le fait d'instaurer plusieurs boucles de circulation permet de diminuer la probabilité du passage de cellules en situation de court-circuit hydraulique, telle que définie ci-dessus.

Le procédé de l'invention comprend également avantageusement une étape d'extraction du produit traité du dispositif de traitement à un débit d'extraction correspondant avantageusement au débit d'alimentation susmentionné.

Il s'entend par étape d'extraction une étape consiste à extraire à la sortie du dispositif en aval de la chambre de traitement du produit, de sorte à éviter une accumulation de produit dans le dispositif. Le produit extrait et traité peut être récupéré dans une cuve de collecte.

Comme mentionné ci-dessus, le dispositif de traitement comprend au moins une chambre de traitement, ce qui signifie qu'il peut en contenir plusieurs.

Dans ce cas, les chambres de traitement peuvent être accolées les unes aux autres, le cycle d'étapes, à savoir respectivement, l'étape d'introduction, l'étape de traitement et l'étape de soutirage telles qu'explicitées ci-dessus, se produisant dans chacune desdites chambres de traitement.

Le procédé de l'invention peut être mis en oeuvre avec un dispositif de traitement comprenant respectivement :
- une unité d'alimentation en produit à traiter, se présentant, par exemple, sous forme d'une cuve, dans laquelle le produit à traiter est contenu ;
- au moins une chambre de traitement émettant un champ électrique pulsé reliée à l'unité d'alimentation via une conduite d'alimentation ;
- une conduite de sortie située en aval de la chambre de traitement ;
- au moins une boucle de circulation dont l'entrée est située en aval de la chambre de traitement et est connectée à la conduite de sortie et la sortie est située en amont de la chambre de traitement et est connectée à la conduite d'alimentation.

Des pompes peuvent être prévues sur la conduite d'alimentation et sur la boucle de circulation.

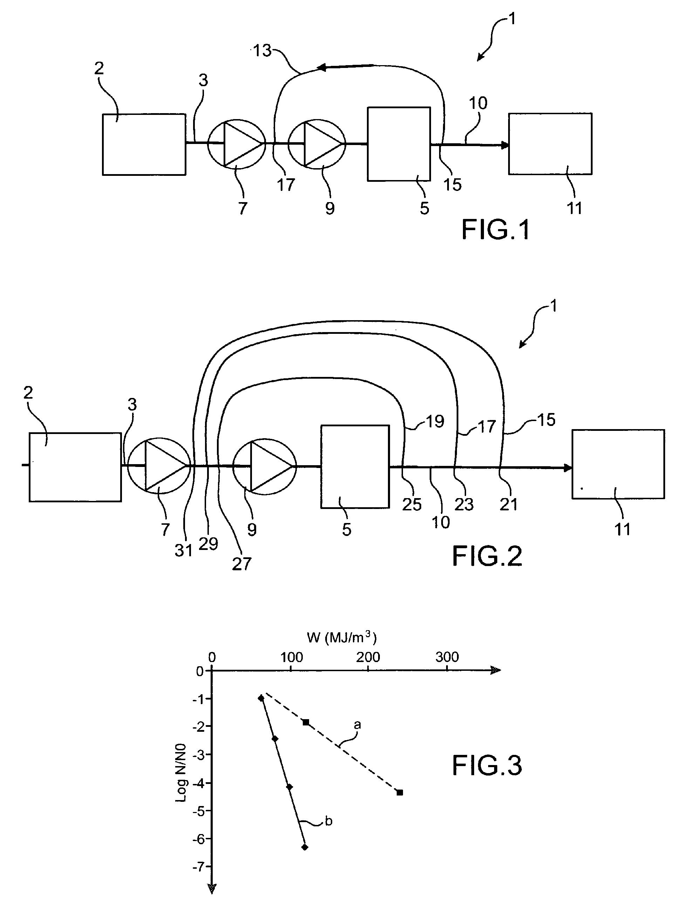

Le procédé de l'invention quand l'étape de soutirage s'effectue via une seule boucle de circulation peut être réalisé à partir d'un dispositif de traitement 1, tel que représenté sur la figure 1, comprenant respectivement :
- une unité d'alimentation 2 comprenant le produit à traiter ;
- une conduite d'alimentation 3 reliant l'unité d'alimentation à une chambre de traitement 5, sur le trajet de laquelle peuvent être interposées des pompes, telles qu'une pompe principale 7 et une pompe secondaire 9 ;
- une chambre de traitement 5 au sein de laquelle est délivré un champ électrique pulsé;
- une conduite de sortie 10 reliant la chambre de traitement 5 à une cuve de réception 11 du liquide traité ;
- une boucle de circulation 13 se matérialisant sous la forme d'une conduite dont l'entrée 15 est située en aval de la chambre de traitement sur la conduite de sortie 10 et la sortie 17 est située en amont de la chambre de traitement 5 sur la conduite d'alimentation 3, cette boucle permettant l'acheminement d'au moins une partie du produit traité en aval de la chambre de traitement afin que cette partie soit de nouveau soumise de nouveau à un champ électrique pulsé.

Le procédé de l'invention, quand l'étape de soutirage s'effectue *via* plusieurs boucles de circulation peut être réalisé à partir d'un dispositif de traitement 1, tel que représenté sur la figure 2, comprenant respectivement :
- une unité d'alimentation 2 comprenant le produit à traiter ;
- une conduite d'alimentation 3 reliant l'unité d'alimentation 3 à une chambre de traitement 5, sur le trajet de laquelle peuvent être interposées des pompes, telles qu'une pompe principale 7 et une pompe secondaire 9 ;
- une chambre de traitement 5 au sein de laquelle est délivré un champ électrique pulsé;
- une conduite de sortie 10 reliant la chambre de traitement 5 à une cuve de réception 11 du liquide traité ;
- trois boucles de circulation respectivement 15, 17 et 19 se matérialisant sous la forme de conduites dont les entrées 21, 23 et 25 sont situées en aval de la chambre de traitement 5 sur la
conduite de sortie 10 et les sorties 27, 29 et 31 sont situées en amont de la chambre de traitement 5 sur la conduite d'alimentation 3, ces boucles permettant l'acheminement d'au moins une partie du produit traité en aval de la chambre de traitement afin que cette partie soit de nouveau soumise à un champ électrique pulsé, éventuellement par le biais de pompe(s) qui peuvent être communes à l'ensemble des boucles de circulation ou indépendante(s) pour chacune des boucles.

Sur la ou les boucles de circulation peuvent être insérés un ou plusieurs appareils tels que des échangeurs de chaleur, des échangeurs de matières (par exemple, des séparateurs de phase), des échangeurs de quantité de mouvement (à savoir, des dispositifs permettant de modifier et améliorer la circulation du fluide, tel qu'une pompe, un agitateur mobile, un agitateur statique).

Le produit à traiter conformément au procédé de l'invention peut se présenter sous forme d'un liquide comprenant des cellules biologiques à perméabiliser, sous forme de boues ou sous forme d'organismes pluricellulaires tels que des fruits.

Lorsqu'il s'agit d'un liquide, il peut s'agir notamment d'eau, d'effluents liquides, des boues liquides de station d'épuration, de jus de fruits, de lait, d'oeufs liquides, de sauces, de soupes, de compotes et de purées.

Il peut s'agir notamment d'un liquide comprenant des organites ou des molécules provenant de cellules biologiques telles que des mitochondries, de l'ADN ou de l'ARN.

Lorsqu'il s'agit de boues, il peut s'agit notamment de boues de station d'épuration.

Le procédé de l'invention peut être destiné à différents usages, tels que :
- la pasteurisation ou la stérilisation de liquides tels que l'eau, les effluents liquides, les jus de fruits, le lait, les oeufs liquides, les sauces, les soupes, les purées, les compotes et les purées ;
- le traitement de boues de station d'épuration, en vue d'éliminer certains organismes pluricellulaires et microorganismes, avant épandage de ces boues ou déshydratation avant séchage ;
- le traitement de cellules biologiques dans le domaine de la manipulation génétique, en vue de les rendre perméables à des molécules exogènes (telles que l'ADN, l'ARN, des protéines, des virus) ;
- l'éclatement de cellules d'organismes pluricellulaires, tels que les fruits, les algues, en vue de faciliter leur pressage ultérieur afin d'obtenir un jus de fruit ou un extrait gras.

Le procédé de l'invention est tout particulièrement adapté à la pasteurisation ou stérilisation d'un liquide.

Ainsi, l'invention a trait également à un procédé de pasteurisation ou stérilisation d'un produit comprenant la mise en oeuvre du procédé tel que défini ci-dessous, les paramètres du champ électrique pulsé étant fixés de sorte à obtenir une perméabilisation membranaire des cellules biologiques présentes dans le liquide conduisant à la mort de ces dernières.

Il peut s'agit notamment de pasteuriser ou stériliser un jus de fruit, comprenant, en tant que cellules biologiques, des levures, telles que *Saccharomyces Cerevisiae.*

L'invention va maintenant être décrite au regard des exemples suivants donnés à titre illustratif et non limitatif.

### BRÈVE DESCRIPTION DES DESSINS

La figure 1 représente un exemple d'un dispositif de traitement comportant une boucle de circulation permettant la mise en oeuvre du procédé de l'invention.
La figure 2 représente un exemple d'un dispositif de traitement comportant trois boucles de circulation permettant la mise en oeuvre du procédé de l'invention.
La figure 3 est un graphique illustrant Log (N/N₀) en fonction de l'énergie spécifique délivrée (en MJ/m³) pour l'exemple comparatif (courbe a) et l'exemple de l'invention (courbe b).

### EXPOSÉ DÉTAILLÉ DE MODES DE RÉALISATION PARTICULIERS

Les exemples suivants illustrent, d'une part, la mise en oeuvre d'un procédé ne rentrant pas dans le cadre de l'invention (dit Exemple Comparatif) et d'un procédé conforme à l'invention (dit Exemple de l'invention).

Pour ce faire, il est utilisé un jus d'orange contaminé par des levures *Saccharomyces cerevisiae.* Ces levures sont caractéristiques des solutions aqueuses riches en sucre et en acides et ont tendance à se multiplier en provoquant un dégagement de CO₂ issu du métabolisme d'oxydation des sucres, lequel phénomène rend le produit impropre à la consommation.

Dans un premier temps, il est procédé à un ensemencement d'un jus d'orange pasteurisé (à savoir, initialement exempt de levures) en ajoutant à ce jus un inoculum concentré, afin de disposer pour les exemples ci-dessous d'un jus enrichi en levures *Saccharomyces cerevisiae* à hauteur de 10⁷ microorganismes par millilitre (dénombrés par une méthode standard de croissance sur milieu spécifique de type gélose).

### Exemple comparatif

Un même volume de jus d'orange (20 litres) est traité une première fois par un dispositif de traitement comprenant une chambre de traitement de volume 5,5 cm³ comprenant des électrodes planes parallèles en inox à un débit de 25 L/h.

Le signal électrique délivré entre les électrodes est un signal à décroissance exponentielle caractéristique de la décharge d'un condensateur dans la résistance électrique que constitue la veine liquide présente dans la chambre. La valeur crête du champ électrique est réglée initialement à une valeur proche de 47 kV/cm produisant une énergie par impulsion proche de 5,5 J/impulsion. La constante de temps τ des impulsions définie par τ=RC est proche de 700 ns, avec R la résistance électrique entre les électrodes et C la valeur de capacité des condensateurs de décharge.

Le jus traité une première fois (nᵣ=1 traitement) est récupéré dans une bonbonne et stocké de façon intermédiaire puis est ensuite traité une seconde fois en le faisant passer, de nouveau, dans le système de traitement, dans les mêmes conditions opératoires (nᵣ=2 traitements) que lors du premier traitement.

Sans tenir compte du délai nécessaire au stockage stagnant intermédiaire, la durée totale du traitement est de 1H36.

Le nombre total d'impulsions (nₜₒₜₐₗ) (correspondant pour chacune à la décharge simple d'un condensateur) auquel est soumis chaque élément unitaire de liquide (lequel élément correspond au volume de la chambre de traitement), lors du premier traitement est égal à 120 et de 120 également lors du second traitement, ce qui porte le nombre final d'impulsions à 240. Les caractéristiques des impulsions sont reportées dans le tableau ci-dessous, à savoir une valeur crête de champ de 46,6 kV/cm et une énergie spécifique délivrée à chaque élément unitaire de liquide ramenée au m³ de 120 MJ/m³, soit 240 MJ/m³ pour les deux traitements.

Les caractéristiques de chaque traitement figurent dans le tableau 1 ci-dessous.

| nᵣ | nₜₒₜₐₗ | Q (L/h) | E (kV/cm) | W_{spéc} (MJ/m³) | Log N/N₀ |
|---|---|---|---|---|---|
| 1 | 120 | 25 | 46, 6 | 120 | -1,87 |
| 2 | 240 | 25 | 46, 6 | 240 | -4,4 |

nᵣ correspondant au nombre de traitement du liquide à traiter ;
nₜₒₜₐₗ correspondant au nombre d'impulsions total auquel est soumis chaque élément unitaire de liquide (lequel élément correspond au volume de la chambre de traitement ;
Q (en L/h) correspondant au débit d'alimentation du liquide;
E (en kV/cm) correspondant à la valeur crête du champ électrique libéré par chacune des impulsions électriques ;
W_{spéc} (en MJ/m³) correspondant à l'énergie spécifique délivrée à chaque élément unitaire de liquide ramenée au m³ ;
Log N/N₀ correspondant au logarithme décimal du rapport du nombre de microorganismes après traitement (N) sur le nombre de microorganismes avant traitement (N₀).

De ce tableau, il apparaît que l'effet de chaque traitement est additif en ce sens que chaque traitement fait décroître le nombre de microorganismes de l'ordre de 2 décades. Par extrapolation, on pourrait considérer que 3 traitements auraient permis d'abaisser de 6 Log la population de microorganismes au prix d'une énergie spécifique de 360 MJ/m³.

### Exemple de l'invention

Un même lot de 20 L jus d'oranges que dans l'exemple comparatif ci-dessus est préparé.

Ce jus alimente le dispositif de traitement à raison de 20 L/h.

Le dispositif de traitement est similaire à celui utilisé pour la mise en oeuvre de l'exemple comparatif exposé ci-dessus si ce n'est qu'il est prévu une boucle de circulation mise en place à la sortie de la chambre de traitement de volume 5,5 cm³. Les caractéristiques de la boucle de circulation sont les suivantes : un diamètre de 8 mm et un volume de 50 mL. Le débit de circulation dans cette boucle est fixé à 360 L/h, ce qui signifie que le débit d'introduction dans la chambre de traitement est de 380 L/h.

Différents essais sont réalisés dans lesquels :
- le même volume de jus est traité (20 L);
- le débit d'alimentation de jus est fixé à 20 L/h tandis que le débit de soutirage de la boucle de circulation est fixé à 360 L/h ;
- la durée de traitement est de 1 heure ;
- la valeur crête du champ électrique est réglée initialement à une valeur proche de 48 kV/cm, produisant une énergie par impulsion proche de 5,8 J/impulsion comparable à la valeur de l'exemple comparatif ci-dessus ;
- le nombre total d'impulsions délivré à chaque élément unitaire de liquide (lequel correspond au volume de la chambre de traitement) à une valeur déterminée différente pour chacun des essais, ce qui correspond à une énergie spécifique donnée.

Les paramètres opératoires des essais mis en oeuvre figurent dans le tableau 2 ci-dessous.

| Essai | nₜₒₜₐₗ | W_{spéc} (en MJ/m³) | N₀ | N | Log (N/N₀) |
|---|---|---|---|---|---|
| 1 | 59 | 62 | 2,2*10⁶ | 2,2*10⁵ | -1 |
| 2 | 79 | 80 | 2,2*10⁶ | 7,75*10³ | -2,45 |
| 3 | 99 | 99 | 2,2*10⁶ | 1,5*10² | -4,17 |
| 4 | 119 | 118 | 2,2*10⁶ | 1 | -6,34 |

nₜₒₜₐₗ correspondant au nombre total d'impulsions délivré à chaque élément unitaire de liquide (lequel correspond au volume de la chambre de traitement) ;
W_{spéc} (en MJ/m³) correspondant à l'énergie spécifique délivrée à chaque élément unitaire de liquide ramenée au m³ ;
N₀ correspondant au nombre de microorganismes avant traitement ;
N correspondant au nombre de microorganismes après traitement ;

Il ressort de ce tableau qu'une inactivation supérieure à 2,4 Log est obtenue pour une énergie spécifique de 80 MJ/m³ (alors que dans le cas de l'exemple comparatif il fallait une énergie spécifique de plus de 120 MJ/m³ pour obtenir une telle inactivation), qu'une inactivation supérieure à 4 Log est obtenue pour une énergie spécifique de 99 MJ/m³ et que, pour une énergie spécifique de 118 MJ/m³, on obtient une inactivation de 6,34 Log (alors que pour une énergie spécifique de 120 MJ/m³, l'on obtenait uniquement une inactivation de l'ordre de 2 Log).

La figure 3 représente un graphique illustrant Log (N/N₀) en fonction de l'énergie spécifique W (en MJ/m³) pour les valeurs obtenues pour l'exemple comparatif (courbe a) et l'exemple de l'invention (courbe b).

En conclusion, il ressort que 4 Log d'inactivation (soit 10 000 fois moins de microorganismes vivants après traitement qu'avant) peuvent être obtenus avec 2,4 fois moins d'énergie dans le cadre de l'exemple de l'invention. Il ressort également que, pour une même valeur d'énergie spécifique (en l'occurrence, 120 MJ/m³) un gain de 4 Log est obtenu dans le cadre de l'exemple de l'invention par rapport à l'exemple comparatif.

L'instauration d'une boucle de circulation permet ainsi d'accéder à des résultats supérieurs à ceux obtenus par des passages multiples du même volume de jus à traiter comme cela est explicité dans l'exemple comparatif.

Ainsi, le procédé de l'invention permet d'envisager une diminution significative à la fois des coûts d'investissement et de fonctionnement tout en diminuant le risque de reviviscence de microorganismes.

Par rapport à un procédé ne comportant pas de boucle de circulation, on minimise le volume mort (c'est-à-dire un volume stagnant de liquide traité, tel que cela est le cas de l'exemple comparatif, où le liquide traité avant son second passage est entreposé dans une cuve intermédiaire).

L'originalité de l'invention provient aussi de l'effet inattendu du procédé, dans lequel la boucle de circulation n'agit pas seulement comme un appareil de mélangeage ou d'augmentation du régime hydraulique.

Dans ce qui suit, on compare la différence de mortalité cellulaire pour des valeurs d'énergie spécifique comparables.

Le produit traité est constitué de jus d'oranges contaminé par des levures similaires à ce qui précède. Le produit est traité dans la même chambre de traitement pour une valeur crête de champ électrique de 48 kV/cm, produisant une énergie par impulsion égale à 5,7 J. Les résultats d'inactivation sont comparés pour une énergie spécifique fournie de 100 MJ/m³ (soit un nombre total d'impulsions de 100).

Dans un premier cas, en l'absence de boucle de circulation avec une charge de 20 L et un débit d'alimentation de 25 L/h, par extrapolation des valeurs de la courbe illustrée par la figure 3, l'inactivation est de 1,5 Log.

Dans un deuxième cas, toujours en l'absence de boucle de circulation, avec une charge de 500 L avec un débit d'alimentation de 500 L/h, l'on obtient une valeur expérimentale Log N/N₀=2 provenant d'un meilleur régime hydrodynamique équivalent à une meilleure agitation.

Si l'on se réfère à la figure 3, avec une charge de 20 L et un débit d'alimentation de 20 L/h, lorsque la boucle de circulation est mise en oeuvre, pour un débit total traversant la chambre de traitement de 380 L/h (360 L/h dans la boucle et 20 L/h provenant de l'alimentation), soit un régime hydraulique légèrement moins favorable qu'à 500 L/h, on obtient une inactivation Log N/N₀ de 5. Donc, l'effet favorable sur la mortalité cellulaire provient donc essentiellement du nombre de passages par la boucle de circulation et non pas du régime hydraulique.

Les résultats figurent dans le tableau 3 ci-après.

| Débit d'alimentation Q (L/h) | Boucle de circulation | Débit d'introduction dans la chambre (en L/h) | W_{spéc} (en MJ/m³) | Log (N/N₀) |
|---|---|---|---|---|
| 25 | Non | 25 | 100 | 1,5 |
| 500 | Non | 500 | 100 | 2 |
| 20 | Oui | 380 | 100 | 5 |

Il pourrait être prévu plusieurs boucles de circulation, par exemple, 2 ou 3 boucles de circulation.

Dans le tableau 2 ci-dessus, on peut observer que, pour une énergie spécifique de 118 MJ/m³, avec une seule boucle de circulation, les microorganismes passent quasiment sans risque de passer avec un court circuit hydraulique, à savoir, dans ce cas, avec une probabilité de passage proche de 1 sur 2*10⁶. Si l'on ajoute une deuxième boucle de circulation, toutes choses égales par ailleurs, la probabilité de passage direct d'un microorganisme sera proche de 1 sur (2*10⁶)², soit 1 sur 4*10¹². Si l'on ajoute une troisième boucle de circulation, la probabilité de passe direct d'un microorganisme sera proche de 1 sur (2*10⁶)³, soit 1 sur 8*10¹⁸.

Ainsi, avec un procédé conforme à l'invention, à partir d'un liquide d'alimentation classiquement contaminé à hauteur de 10⁶ à 10⁷ microorganismes par millilitre, l'on peut ainsi quasiment annuler toute probabilité de passage d'un microorganisme en court circuit hydraulique et, donc se prémunir de tous risques de reviviscence d'un liquide pasteurisé par champs électriques pulsés.

## Revendications

1. Procédé de perméabilisation membranaire de cellules biologiques contenues dans un produit, ledit procédé étant mis en oeuvre dans un dispositif de traitement comprenant au moins une chambre de traitement émettant un champ électrique pulsé, ledit procédé comprenant les étapes suivantes:
- une étape d'alimentation du dispositif de traitement en produit comprenant lesdites cellules biologiques à un débit d'alimentation prédéterminé à partir d'une unité d'alimentation comprenant ledit produit ;
- une étape d'introduction du produit comprenant lesdites cellules biologiques dans ladite chambre de traitement à un débit d'introduction correspondant au débit d'alimentation susmentionné auquel s'ajoute le débit de soutirage mentionné à l'étape de soutirage ci-dessous ;
- une étape de traitement dudit produit introduit dans ladite chambre par un champ électrique pulsé ;
- une étape de soutirage, à un débit de soutirage prédéterminé, du produit en sortie de ladite chambre de sorte à le réacheminer en amont de ladite chambre et en aval de ladite unité d'alimentation.

2. Procédé selon la revendication 1, dans lequel les cellules biologiques sont choisies parmi les cellules procaryotes, les cellules eucaryotes, ces cellules pouvant être vivantes ou mortes, entières ou partielles et d'origine animale ou végétale.

3. Procédé selon l'une quelconque des revendications précédentes, dans lequel les cellules biologiques sont issues d'organismes unicellulaires ou pluricellulaires choisies parmi les bactéries, les champignons, les levures, les moisissures, les algues.

4. Procédé selon l'une quelconque des revendications précédentes, dans lequel le champ électrique pulsé se matérialise sous forme d'impulsions électriques résultant de décharges électriques de durée pouvant s'échelonner de 50 ns à 1 ms et délivrant une tension produisant une valeur crête du champ électrique pulsé allant de 5 kV/cm à 200 kV/cm.

5. Procédé selon l'une quelconque des revendications précédentes, dans lequel le produit, notamment lorsqu'il se présente sous forme d'un liquide, est soumis à un régime hydraulique turbulent.

6. Procédé selon l'une quelconque des revendications précédentes, dans lequel l'étape de soutirage est réalisée par le biais d'au moins une boucle de circulation reliant la partie aval de la chambre de traitement à la partie amont de celle-ci.

7. Procédé selon l'une quelconque des revendications précédentes, dans lequel le débit de soutirage est supérieur au débit d'alimentation.

8. Procédé selon la revendication 7, dans lequel le débit de soutirage est de 2 à 100 fois plus élevé que le débit d'alimentation.

9. Procédé selon la revendication 7 ou 8, dans lequel le débit de soutirage est de 2 à 20 fois plus élevé que le débit d'alimentation.

10. Procédé selon l'une quelconque des revendications précédentes, ne comprenant pas d'étape de stockage stagnant du produit traité avant l'étape de soutirage.

11. Procédé selon l'une quelconque des revendications précédentes, comprenant également avantageusement une étape d'extraction du produit traité du dispositif de traitement à un débit d'extraction correspondant, de préférence, au débit d'alimentation susmentionné.

12. Procédé selon l'une quelconque des revendications précédentes, dans lequel le produit à traiter est un liquide comprenant des cellules biologiques, une boue contenant des cellules biologiques ou un organisme pluricellulaire tel qu'un fruit.

13. Procédé selon l'une quelconque des revendications précédentes, dans lequel le produit à traiter est un liquide comprenant des organites ou des molécules provenant de cellules biologiques, telles que des mitochondries, de l'ADN, de l'ARN.

14. Procédé selon la revendication 12 ou 13, dans lequel le liquide est choisi parmi l'eau, les effluents liquides, les boues liquides de station d'épuration, les jus de fruits, le lait, les oeufs liquides, les sauces, les soupes, les compotes et les purées.

15. Procédé de pasteurisation ou de stérilisation d'un produit comprenant des cellules biologiques à éliminer comprenant une étape de mise en oeuvre du procédé tel que défini selon l'une quelconque des revendications 1 à 14.

16. Procédé selon la revendication 15, dans lequel le produit est un jus de fruits comprenant, en tant que cellules biologiques, des levures, telles que *Saccharomyces Cerevisiae.*

## Claims

1. A method for membrane permeabilization of biological cells contained in a product, said method being applied in a treatment device comprising at least one treatment chamber emitting a pulsed electric field, said method comprising the following steps:
- a step for supplying the treatment device with a product comprising said biological cells at a predetermined supply flow rate from a supply unit comprising said product;
- a step for introducing the product comprising said biological cells in said treatment chamber at an introduction flow rate corresponding to the aforementioned supply flow rate to which is added the drawing-off of flow rate mentioned in the drawing-off step below;
- a step for treating said product introduced into said chamber with a pulsed electric field;
- a drawing-off step, at a predetermined drawing-off flow rate, for the product at the outlet of said chamber so as to transport it back upstream from said chamber and downstream from said supply unit.

2. The method according to claim 1, wherein the biological cells are selected from prokaryotic cells, eukaryotic cells, these cells may be live or dead, entire or partial or cells of animal or vegetable origin.

3. The method according to any of the preceding claims, wherein the biological cells stem from unicellular or pluricellular organisms selected from bacteria, fungi, yeasts, molds, algae.

4. The method according to any of the preceding claims, wherein the pulsed electric field is materialized as electric pulses resulting from electric discharges with a duration which may range from 50 nanoseconds to 1 millisecond and delivering a voltage producing a peak value of the pulsed electric field ranging from 5 kV/cm to 200 kV/cm.

5. The method according to any of the preceding claims, wherein the product, notably when it appears as a liquid, is subject to turbulent hydraulic conditions.

6. The method according to any of the preceding claims, wherein the drawing-off step is carried out by means of at least one circulation loop connecting the downstream portion of the treatment chamber to the upstream portion of the latter.

7. The method according to any of the preceding claims, wherein the drawing-off flow rate is greater than the supply flow rate.

8. The method according to claim 7, wherein the drawing-off flow rate is from 2 to 100 times greater than the supply flow rate.

9. The method according to claim 7 or 8, wherein the drawing-off flow rate is 2 to 20 times greater than the supply flow rate.

10. The method according to any of the preceding claims, not comprising any stagnant storage step for the treated product before the drawing-off step.

11. The method according to any of the preceding claims, also advantageously comprising a step for extracting the treated product from the treatment device at an extraction flow rate corresponding preferably to the aforementioned supply flow rate.

12. The method according to any of the preceding claims, wherein the product to be treated is a liquid comprising biological cells, a mud containing biological cells or a pluricellular organism such as fruit.

13. The method according to any of the preceding claims, wherein the product to be treated is a liquid comprising organites or molecules from biological cells such as mitochondria, DNA, RNA.

14. The method according to claim 12 or 13, wherein the liquid is selected from water, liquid effluents, liquid muds from sewage works, fruit juices, milk, liquid eggs, sauces, soups, stewed fruit and purees.

15. A method for pasteurizing or sterilizing a product comprising biological cells to be removed comprising a step for applying the method as defined according to any of claims 1 to 14.

16. The method according to claim 15, wherein the product is a fruit juice comprising as biological cells, yeasts such as *Saccharomyces Cerevisiae.*

## Patentansprüche

1. Verfahren zum Durchlässigmachen der Membran von biologischen Zellen, die in einem Produkt enthalten sind, wobei das Verfahren in einer Behandlungsvorrichtung durchgeführt wird, die zumindest eine Behandlungskammer aufweist, die ein gepulstes, elektrisches Feld emittiert, wobei das Verfahren die nachfolgenden Schritte umfasst:
- einen Schritt des Versorgens der Behandlungsvorrichtung mit dem Produkt, das die biologischen Zellen enthält, und zwar mit einer vorbestimmten Versorgungsdurchsatzmenge ausgehend von einer das Produkt enthaltenden Versorgungseinheit;
- einem Schritt des Einführens des die biologischen Zellen enthaltenden Produkts in die Behandlungskammer mit einer Einführdurchsatzmenge, die der genannten Versorgungsdurchsatzmenge entspricht, zu der die in dem nachfolgenden Schritt des Abziehens genannte Abziehdurchsatzmenge hinzukommt;
- einen Schritt des Behandelns des in die Kammer eingeführen Produkts mit einem gepulsten, elektrischen Feld;
- einen Schritt des Abziehens des Produkts mit einer vorbestimmten Abziehdurchsatzmenge am Austritt aus der Kammer, so dass es stromaufwärts der Kammer und stromabwärts der Versorgungseinheit weitergeleitet wird.

2. Verfahren nach Anspruch 1, wobei die biologischen Zellen ausgewählt sind aus procaryotischen Zellen, eucaryotischen Zellen, wobei diese Zellen lebend oder tot sein können, ganze Zellen oder Teilzellen und von tierischer oder pflanzlicher Herkunft sein können.

3. Verfahren nach einem der vorangehenden Ansprüche, wobei die biologischen Zellen aus Einzellern oder mehrzelligen Organismen strammen, die ausgewählt sind aus Bakterien, Pilzen, Hefen, Schimmelpilzen, Algen.

4. Verfahren nach einem der vorangehenden Ansprüche, wobei das gepulste elektrische Feld in Form von elektrischen Impulsen auftritt, die sich aus elektrischen Entladungen mit einer Zeitdauer, die von 50 ns bis 1 ms gestaffelt sein kann, ergeben und eine Spannung liefern, die einen Spitzenwert des gepulsten elektrischen Feldes erzeugt, der von 5 kV/cm bis 200 kV/cm reicht.

5. Verfahren nach einem der vorangehenden Ansprüche, wobei das Produkt insbesondere dann, wenn es in Form einer Flüssigkeit vorliegt, einer turbulenten hydraulischen Strömung unterworfen wird.

6. Verfahren nach einem der vorangehenden Ansprüche, wobei der Schritt des Abziehens über zumindest einen Zirkulationskreis erfolgt, welcher den stromabwärtigen Teil der Behandlungskammer mit dem stromaufwärtigen Teil derselben verbindet.

7. Verfahren nach einem der vorangehenden Ansprüche, wobei die Abziehdurchsatzmenge größer ist als die Versorgungsdurchsatzmenge.

8. Verfahren nach Anspruch 7, wobei die Abziehdurchsatzmenge um 2- bis 100-mal höher ist als die Versorgungsdurchsatzmenge.

9. Verfahren nach Anspruch 7 oder 8, wobei die Abziehdurchsatzmenge um 2-bis 20-mal höher ist als die Versorgungsdurchsatzmenge.

10. Verfahren nach einem der vorangehenden Ansprüche, wobei es keinen Schritt der stillstehenden Speicherung des behandelten Produkts vor dem Schritt des Abziehens umfasst.

11. Verfahren nach einem der vorangehenden Ansprüche, wobei es auch einen Schritt des Abführens des behandelten Produkts aus der Behandlungsvorrichtung mit einer entsprechenden Abführdurchsatzmenge, vorzugsweise mit der genannten Versorgungsdurchsatzmenge umfasst.

12. Verfahren nach einem der vorangehenden Ansprüche, wobei das zu behandelnde Produkt eine Flüssigkeit mit biologischen Zellen, ein Schlamm mit biologischen Zellen oder ein mehrzelliger Organismus, wie etwa eine Frucht, ist.

13. Verfahren nach einem der vorangehenden Ansprüche, wobei das zu behandelnde Produkt eine Flüssigkeit mit Organellen oder Molekülen aus biologischen Zellen, wie etwa Mitochondrien, DNA, RNA, ist.

14. Verfahren nach Anspruch 12 oder 13, wobei die Flüssigkeit ausgewählt ist aus Wasser, Abwässern, flüssigen Schlämmen aus Kläranlagen, Fruchtsäften, Milch, flüssigen Eiern, Soßen, Suppen, Kompotten und Pürees.

15. Verfahren zum Pasteurisieren oder Sterilisieren eines Produkts mit biologischen Zellen, die zu beseitigen sind, mit einem Schritt des Durchführens des Verfahrens wie nach einem der Ansprüche 1 bis 14 definiert.

16. Verfahren nach Anspruch 15, wobei das Produkt ein Fruchtsaft ist, der als biologische Zellen Hefen, wie etwa *Saccharomyces Cerevisiae,* enthält.
